# EUROPEAN PATENT SPECIFICATION

(11) **EP 2 243 033 B1**
(45) Date of publication and mention of the grant of the patent: **06.06.2012**
(21) Application number: 09708303.4
(22) Date of filing: 29.01.2009
(51) Int. Cl.: G01N 33/68

(54) **THERMAL DENATURATION SCREENING ASSAY TO IDENTIFY CANDIDATE COMPOUNDS FOR PREVENTION AND TREATMENT OF PARKINSON'S DISEASE**
THERMISCHER DENATURIERUNGSSCREENINGTEST ZUR IDENTIFIZIERUNG VON VERBINDUNGSKANDIDATEN ZUR PRÄVENTION UND BEHANDLUNG VON MORBUS PARKINSON
ANALYSE DE CRIBLAGE PAR DÉNATURATION THERMIQUE POUR IDENTIFIER DES COMPOSÉS CANDIDATS POUR LA PRÉVENTION ET LE TRAITEMENT DE LA MALADIE DE PARKINSON

(30) Priority: 31.01.2008 US 25231 P
(43) Date of publication of application: 27.10.2010
(73) Proprietor: Elan Pharma International Limited, County Westmeath (IE)
(72) Inventor: JOHNSON, Jennifer, A., Mill Valley California 94941 (US); SZOKE, Balazs, G., San Carlos California 94070 (US); MCCONLOGUE, Lisa, Burlingame California 94010 (US)
(74) Representative: Bullett, Rachel Margaret
(86) International application number: PCT/US2009/032473
(87) International publication number: WO 2009/099896

(56) References cited:
- WO-A-2007/089334
- WO-A-2008/095126
- WO-A-2008/144736
- WINKLHOFER KONSTANZE F ET AL: "Inactivation of parkin by oxidative stress and C-terminal truncations: a protective role of molecular chaperones." THE JOURNAL OF BIOLOGICAL CHEMISTRY 21 NOV 2003, vol. 278, no. 47, 21 November 2003 (2003-11-21), pages 47199-47208, XP002527803 ISSN: 0021-9258
- SAFADI SUSAN S ET AL: "A disease state mutation unfolds the parkin ubiquitin-like domain." BIOCHEMISTRY 11 DEC 2007, vol. 46, no. 49, 11 December 2007 (2007-12-11), pages 14162-14169, XP002527804 ISSN: 0006-2960
- VAN HUMBEECK CINDY ET AL: "Parkin occurs in a stable, non-covalent, approximately 110-kDa complex in brain." THE EUROPEAN JOURNAL OF NEUROSCIENCE JAN 2008, vol. 27, no. 2, 27 January 2008 (2008-01-27), pages 284-293, XP002527743 ISSN: 1460-9568
- MURATA SHIGEO ET AL: "Purification and assay of the chaperone-dependent ubiquitin ligase of the carboxyl terminus of Hsc70-interacting protein" UBIQUITIN AND PROTEIN DEGRADATION, PART A, METHODS IN ENZYMOLOGY, ELSEVIER INC, vol. 398, 1 January 2005 (2005-01-01), pages 271-279, XP008106050 ISBN: 978-0-12-182803-5 [retrieved on 2005-11-04]

### SEQUENCE LISTING

<110> Johnston, Jennifer A.
   Szoke, Balazs G.
   McConlogue, Lisa
<120> THERMAL DENATURATION SCREENING ASSAY TO
   IDENTIFY CANDIDATE COMPOUNDS FOR PREVENTION AND TREATMENT OF PARKINSON'S DISEASE
<130> 015270-022710US
<140> Not yet assigned
   <141> 2009-01-29
<150> US 61/025,231
   <151> 2008-01-31
<160> 8
<170> FastSEQ for Windows Version 4.0
<210> 1
   <211> 1398
   <212> DNA
   <213> Homo sapiens
<220>
   <223> polynucleotide encoding Parkin
<400> 1
   atgatagtgt ttgtcaggtt caactccagc catggtttcc cagtggaggt cgattctgac 60
   accagcatct tccagctcaa ggaggtggtt gctaagcgac agggggttcc ggctgaccag 120
   ttgcgtgtga ttttcgcagg gaaggagctg aggaatgact ggactgtgca gaattgtgac 180
   ctggatcagc agagcattgt tcacattgtg cagagaccgt ggagaaaagg tcaagaaatg 240
   aatgcaactg gaggcgacga ccccagaaac gcggcgggag gctgtgagcg ggagccccag 300
   agcttgactc gggtggacct cagcagctca gtcctcccag gagactctgt ggggctggct 360
   gtcattctgc acactgacag caggaaggac tcaccaccag ctggaagtcc agcaggtaga 420
   tcaatctaca acagctttta tgtgtattgc aaaggcccct gtcaaagagt gcagccggga 480
   aaactcaggg tacagtgcag cacctgcagg caggcaacgc tcaccttgac ccagggtcca 540
   tcttgctggg atgatgtttt aattccaaac cggatgagtg gtgaatgcca atccccacac 600
   tgccctggga ctagtgcaga atttttcttt aaatgtggag cacaccccac ctctgacaag 660
   gaaacaccag tagctttgca cctgatcgca acaaatagtc ggaacatcac ttgcattacg 720
   tgcacagacg tcaggagccc cgtcctggtt ttccagtgca actcccgcca cgtgatttgc 780
   ttagactgtt tccacttata ctgtgtgaca agactcaatg atcggcagtt tgttcacgac 840
   cctcaacttg gctactccct gccttgtgtg gctggctgtc ccaactcctt gattaaagag 900
   ctccatcact tcaggattct gggagaagag cagtacaacc ggtaccagca gtatggtgca 960
   gaggagtgtg tcctgcagat ggggggcgtg ttatgccccc gccctggctg tggagcgggg 1020
   ctgctgccgg agcctgacca gaggaaagtc acctgcgaag ggggcaatgg cctgggctgt 1080
   gggtttgcct tctgccggga atgtaaagaa gcgtaccatg aaggggagtg cagtgccgta 1140
   tttgaagcct caggaacaac tactcaggcc tacagagtcg atgaaagagc cgccgagcag 1200
   gctcgttggg aagcagcctc caaagaaacc atcaagaaaa ccaccaagcc ctgtccccgc 1260
   tgccatgtac cagtggaaaa aaatggaggc tgcatgcaca tgaagtgtcc gcagccccag 1320
   tgcaggctcg agtggtgctg gaactgtggc tgcgagtgga accgcgtctg catgggggac 1380
   cactggttcg acgtgtag 1398
<210> 2
   <211> 465
   <212> PRT
   <213> Homo sapiens
<220>
   <223> Parkin
<400> 2
   Met Ile Val Phe Val Arg Phe Asn Ser Ser His Gly Phe Pro Val Glu
    1               5                  10                  15
   Val Asp Ser Asp Thr Ser Ile Phe Gln Leu Lys Glu Val Val Ala Lys
               20                  25                  30
   Arg Gln Gly Val Pro Ala Asp Gln Leu Arg Val Ile Phe Ala Gly Lys
           35                  40                  45
   Glu Leu Arg Asn Asp Trp Thr Val Gln Asn Cys Asp Leu Asp Gln Gln
       50                  55                  60
   Ser Ile Val His Ile Val Gln Arg Pro Trp Arg Lys Gly Gln Glu Met
   65                  70                  75                  80
   Asn Ala Thr Gly Gly Asp Asp Pro Arg Asn Ala Ala Gly Gly Cys Glu
                   85                  90                  95
   Arg Glu Pro Gln Ser Leu Thr Arg Val Asp Leu Ser Ser Ser Val Leu
               100                 105                 110
   Pro Gly Asp Ser Val Gly Leu Ala Val Ile Leu His Thr Asp Ser Arg
           115                 120                 125
   Lys Asp Ser Pro Pro Ala Gly Ser Pro Ala Gly Arg Ser Ile Tyr Asn
       130                 135                 140
   Ser Phe Tyr Val Tyr Cys Lys Gly Pro Cys Gln Arg Val Gln Pro Gly
   145                 150                 155                 160
   Lys Leu Arg Val Gln Cys Ser Thr Cys Arg Gln Ala Thr Leu Thr Leu
                   165                 170                 175
   Thr Gln Gly Pro Ser Cys Trp Asp Asp Val Leu Ile Pro Asn Arg Met
               180                 185                 190
   Ser Gly Glu Cys Gln Ser Pro His Cys Pro Gly Thr Ser Ala Glu Phe
           195                 200                 205
   Phe Phe Lys Cys Gly Ala His Pro Thr Ser Asp Lys Glu Thr Pro Val
       210                 215                 220
   Ala Leu His Leu Ile Ala Thr Asn Ser Arg Asn Ile Thr Cys Ile Thr
   225                 230                 235                 240
   Cys Thr Asp Val Arg Ser Pro Val Leu Val Phe Gln Cys Asn Ser Arg
                   245                 250                 255
   His Val Ile Cys Leu Asp Cys Phe His Leu Tyr Cys Val Thr Arg Leu
               260                 265                 270
   Asn Asp Arg Gln Phe Val His Asp Pro Gln Leu Gly Tyr Ser Leu Pro
           275                 280                 285
   Cys Val Ala Gly Cys Pro Asn Ser Leu Ile Lys Glu Leu His His Phe
       290                 295                 300
   Arg Ile Leu Gly Glu Glu Gln Tyr Asn Arg Tyr Gln Gln Tyr Gly Ala
   305                 310                 315                 320
   Glu Glu Cys Val Leu Gln Met Gly Gly Val Leu Cys Pro Arg Pro Gly
                   325                 330                 335
   Cys Gly Ala Gly Leu Leu Pro Glu Pro Asp Gln Arg Lys Val Thr Cys
               340                 345                 350
   Glu Gly Gly Asn Gly Leu Gly Cys Gly Phe Ala Phe Cys Arg Glu Cys
           355                 360                 365
   Lys Glu Ala Tyr His Glu Gly Glu Cys Ser Ala Val Phe Glu Ala Ser
       370                 375                 380
   Gly Thr Thr Thr Gln Ala Tyr Arg Val Asp Glu Arg Ala Ala Glu Gln
   385                 390                 395                 400
   Ala Arg Trp Glu Ala Ala Ser Lys Glu Thr Ile Lys Lys Thr Thr Lys
                   405                 410                 415
   Pro Cys Pro Arg Cys His Val Pro Val Glu Lys Asn Gly Gly Cys Met
               420                 425                 430
   His Met Lys Cys Pro Gln Pro Gln Cys Arg Leu Glu Trp Cys Trp Asn
           435                 440                 445
   Cys Gly Cys Glu Trp Asn Arg Val Cys Met Gly Asp His Trp Phe Asp
       450                 455                 460
   Val
   465
<210> 3
   <211> 1395
   <212> DNA
   <213> Mus musculus
<220>
   <223> polynucleotide encoding Parkin
<400> 3
   atgatagtgt ttgtcaggtt caactccagc tatggcttcc cagtggaggt cgattctgac 60
   accagcatct tgcagctcaa ggaagtggtt gctaagcgac agggggttcc agctgaccag 120
   ctgcgtgtga tttttgccgg gaaggagctt ccgaatcacc tgacggttca aaactgtgac 180
   ctggaacaac agagtattgt acacatagta cagagaccac ggaggagaag tcatgaaaca 240
   aatgcatctg gaggggacga accccagagc acctcagagg gctccatatg ggagtccagg 300
   agcttgacac gagtggacct gagcagccat accctgccgg tggactctgt ggggctggcg 360
   gtcattctgg acacagacag taagagggat tcagaagcag ccagaggtcc agttaaaccc 420
   acctacaaca gctttttcat ctactgcaaa ggcccctgcc acaaggtcca gcctggaaag 480
   ctccgagttc agtgtggcac ctgcaaacaa gcaaccctca ccttggccca gggcccatct 540
   tgctgggacg atgtcttaat tccaaaccgg atgagtggtg agtgccagtc tccagactgc 600
   cctggaacca gagctgaatt tttctttaaa tgtggagcac acccaacctc agacaaggac 660
   acgtcggtag ctttgaacct gatcaccagc aacaggcgca gcatcccttg catagcgtgc 720
   acagatgtca ggagccctgt cctggtcttc cagtgtaacc accgtcacgt gatctgtttg 780
   gactgtttcc acttgtattg tgtcacaaga ctcaacgatc ggcagtttgt ccacgatgct 840
   caacttggct actccctgcc gtgtgtagct ggctgtccca actccctgat taaagagctc 900
   catcacttca ggatccttgg agaagagcag tacactaggt accagcagta tggggccgag 960
   gaatgcgtgc tgcaaatggg aggtgtgctg tgcccccgtc ctggctgtgg agctggactg 1020
   ctacctgaac agggccagag gaaagtcacc tgcgaagggg gcaacggcct gggctgcggg 1080
   tttgttttct gccgggactg taaggaagca taccatgaag gggattgcga ctcactgctc 1140
   gaaccctcag gagccacttc tcaggcctac agggtggaca aaagagccgc tgagcaagct 1200
   cgctgggagg aggcctccaa ggaaaccatc aagaagacca ccaagccttg tcctcgctgc 1260
   aacgtgccaa ttgaaaaaaa cggaggatgt atgcacatga agtgtcctca gccccagtgc 1320
   aagctggagt ggtgctggaa ctgtggctgt gagtggaacc gagcctgcat gggagatcac 1380
   tggtttgacg tgtag                                                  1395
<210> 4
   <211> 464
   <212> PRT
   <213> Mus musculus
<220>
   <223> Parkin
<400> 4
   Met Ile Val Phe Val Arg Phe Asn Ser Ser Tyr Gly Phe Pro Val Glu
   1               5                   10                  15
   Val Asp Ser Asp Thr Ser Ile Leu Gln Leu Lys Glu Val Val Ala Lys
               20                  25                  30
   Arg Gln Gly Val Pro Ala Asp Gln Leu Arg Val Ile Phe Ala Gly Lys
           35                  40                  45
   Glu Leu Pro Asn His Leu Thr Val Gln Asn Cys Asp Leu Glu Gln Gln
       50                  55                  60
   Ser Ile Val His Ile Val Gln Arg Pro Arg Arg Arg Ser His Glu Thr
   65                  70                  75                  80
   Asn Ala Ser Gly Gly Asp Glu Pro Gln Ser Thr Ser Glu Gly Ser Ile
                       85                  90                  95
   Trp Glu Ser Arg Ser Leu Thr Arg Val Asp Leu Ser Ser His Thr Leu
               100                 105                 110
   Pro Val Asp Ser Val Gly Leu Ala Val Ile Leu Asp Thr Asp Ser Lys
           115                 120                 125
   Arg Asp Ser Glu Ala Ala Arg Gly Pro Val Lys Pro Thr Tyr Asn Ser
       130                 135                 140
   Phe Phe Ile Tyr Cys Lys Gly Pro Cys His Lys Val Gln Pro Gly Lys
   145                 150                 155                 160
   Leu Arg Val Gln Cys Gly Thr Cys Lys Gln Ala Thr Leu Thr Leu Ala
                   165                 170                 175
   Gln Gly Pro Ser Cys Trp Asp Asp Val Leu Ile Pro Asn Arg Met Ser
               180                 185                 190
   Gly Glu Cys Gln Ser Pro Asp Cys Pro Gly Thr Arg Ala Glu Phe Phe
           195                 200                 205
   Phe Lys Cys Gly Ala His Pro Thr Ser Asp Lys Asp Thr Ser Val Ala
       210                 215                 220
   Leu Asn Leu Ile Thr Ser Asn Arg Arg Ser Ile Pro Cys Ile Ala Cys
   225                 230                 235                 240
   Thr Asp Val Arg Ser Pro Val Leu Val Phe Gln Cys Asn His Arg His
                   245                 250                 255
   Val Ile Cys Leu Asp Cys Phe His Leu Tyr Cys Val Thr Arg Leu Asn
               260                 265                 270
   Asp Arg Gln Phe Val His Asp Ala Gln Leu Gly Tyr Ser Leu Pro Cys
           275                 280                 285
   Val Ala Gly Cys Pro Asn Ser Leu Ile Lys Glu Leu His His Phe Arg
       290                 295                 300
   Ile Leu Gly Glu Glu Gln Tyr Thr Arg Tyr Gln Gln Tyr Gly Ala Glu
   305                 310                 315                 320
   Glu Cys Val Leu Gln Met Gly Gly Val Leu Cys Pro Arg Pro Gly Cys
                   325                 330                 335
   Gly Ala Gly Leu Leu Pro Glu Gln Gly Gln Arg Lys Val Thr Cys Glu
               340                 345                 350
   Gly Gly Asn Gly Leu Gly Cys Gly Phe Val Phe Cys Arg Asp Cys Lys
           355                 360                 365
   Glu Ala Tyr His Glu Gly Asp Cys Asp Ser Leu Leu Glu Pro Ser Gly
       370                 375                 380
   Ala Thr Ser Gln Ala Tyr Arg Val Asp Lys Arg Ala Ala Glu Gln Ala
   385                 390                 395                 400
   Arg Trp Glu Glu Ala Ser Lys Glu Thr Ile Lys Lys Thr Thr Lys Pro
                   405                 410                 415
   Cys Pro Arg Cys Asn Val Pro Ile Glu Lys Asn Gly Gly Cys Met His
               420                 425                 430
   Met Lys Cys Pro Gln Pro Gln Cys Lys Leu Glu Trp Cys Trp Asn Cys
           435                 440                 445
   Gly Cys Glu Trp Asn Arg Ala Cys Met Gly Asp His Trp Phe Asp Val
       450                 455                 460
<210> 5
   <211> 6
   <212> PRT
   <213> Artificial Sequence
<220>
   <223> synthetic polyhistidine tag
<400> 5
   His His His His His His
   1               5
<210> 6
   <211> 1527
   <212> DNA
   <213> Homo sapiens
<220>
   <223> polynucleotide encoding S5a
<400> 6
   aattggagga gttgttgtta ggccgtcccg gagacccggt cgggagggag gaaggtggca 60
   agatggtgtt ggaaagcact atggtgtgtg tggacaacag tgagtatatg cggaatggag 120
   acttcttacc caccaggctg caggcccagc aggatgctgt caacatagtt tgtcattcaa 180
   agacccgcag caaccctgag aacaacgtgg gccttatcac actggctaat gactgtgaag 240
   tgctgaccac actcacccca gacactggcc gtatcctgtc caagctacat actgtccaac 300
   ccaagggcaa gatcaccttc tgcacgggca tccgcgtggc ccatctggct ctgaagcacc 360
   gacaaggcaa gaatcacaag atgcgcatca ttgcctttgt gggaagccca gtggaggaca 420
   atgagaagga tctggtgaaa ctggctaaac gcctcaagaa ggagaaagta aatgttgaca 480
   ttatcaattt tggggaagag gaggtgaaca cagaaaagct gacagccttt gtaaacacgt 540
   tgaatggcaa agatggaacc ggttctcatc tggtgacagt gcctcctggg cccagtttgg 600
   ctgatgctct catcagttct ccgattttgg ctggtgaagg tggtgccatg ctgggtcttg 660
   gtgccagtga ctttgaattt ggagtagatc ccagtgctga tcctgagctg gccttggccc 720
   ttcgtgtatc tatggaagag cagcggcagc ggcaggagga ggaggcccgg cgggcagctg 780
   cagcttctgc tgctgaggcc gggattgcta cgactgggac tgaaggtgaa agaggtggaa 840
   tccgaagtcc tgggactgcg ggatgctaaa cattgaaagc tgggtgtagg cactgcaggg 900
   agagtgtgga ggtctgacag ggtaggaata tgtgggaggg ctgggctagg aatggccttg 960
   gaggctggcc tgtgtggata tggcaccaat tctaccctgc tcctcttttc cttttcccag 1020
   actcagacga tgccctgctg aagatgacca tcagccagca agagtttggc cgcactgggc 1080
   ttcctgacct aagcagtatg actgaggaag agcagattgc ttatgccatg cagatgtccc 1140
   tgcagggagc agagtttggc caggcggaat cagcagacat tgatgccagc tcagctatgg 1200
   acacatctga gccagccaag gaggaggatg attacgacgt gatgcaggac cccgagttcc 1260
   ttcagagtgt cctagagaac ctcccaggtg tggatcccaa caatgaagcc attcgaaatg 1320
   ctatgggctc cctggcctcc caggccacca aggacggcaa gaaggacaag aaggaggaag 1380
   acaagaagtg agactggagg gaaagggtag ctgagtctgc ttaggggact gcatgggaag 1440
   cacggaatat agggttagat gtgtgttatc tgtaaccatt acagcctaaa taaagcttgg 1500
   caactttttt tccttttttg cttcaaa                                     1527
<210> 7
   <211> 377
   <212> PRT
   <213> Homo sapiens
<220>
   <223> human S5a
<400> 7
<210> 8
   <211> 210
   <212> PRT
   <213> Homo sapiens
<220>
   <223> Troponin 1
<400> 8

## Description

### Field

Screening assays are provided to identify agents for treatment of Parkinson's Disease. The invention has application in the fields of medicine and drug development.

### Background

Parkinson's disease (PD) is a neurological disorder characterized neuro-pathologically as a loss of dopamine neurons of the substantia nigra. This neuronal loss manifests clinically as alterations in movement, such as Bradykinesia, rigidity and/or tremor (Gelb et al., Arch. Neurol., 56:33-39, (1999)). Human genetic data have identified genes linked to the development of PD. One of these genes was localized to chromosome 6 using a cohort of juvenile onset patients and identified as Parkin protein (Kitada et al., Nature. 392:605-608 (1998)). Parkin protein is an E3 ligase protein that functions in the ubiquitin-proteasome pathway (UPS) (Shimura, Nature Genetics, 25:302-305 (2000)). The UPS is a major cellular pathway involved in the targeted removal of proteins for degradation and E3 ligases function to identify and label substrates for degradation by cellular proteasomes (Hereshko et al., Ann. Rev. Biochem., 67:425-479 (1998)) or lysosomes (Hicke, Trends in Cell Biology, 9:107-112 (1999)).

Another hallmark of PD is the presence of insoluble proteinaceous cellular inclusions known as Lewy Bodies. Lewy Bodies are comprised of many proteins, the most prominent being the α-synuclein protein (Spillantini et al., Nature, 388:839-40 (1997)). Point mutations in the α-synuclein gene or multiplications of the gene, result in PD (Polymeropoulos et al., Science, 276:2045-7 (1997); Kruger et al., Nature Genetics, 18:106-8 (1998)).
WO-A-2007/089334 discloses assays for identification of, or screening for, compounds useful for treatment of PD, including cell-based assays for agents that modulate the effect of Parkin proteins on proteasome function.

New therapeutic agents for treating Parkinson's disease are urgently needed. The present invention provides new methods and materials useful for identifying and validating agents that modulate parkin activity, including new therapeutic agents.

### Brief Summary of the Invention

The invention provides an *in vitro* screening assay to identify candidate compounds for prevention and treatment Parkinson's Disease. Parkin protein ("parkin") is exposed to conditions ("thermal destabilization conditions") that cause loss of parkin ligase activity. The exposure to thermal destabilization conditions is carried out in the presence or absence of test agents. Agents that preserve parkin ligase activity are candidate compounds for treatment of Parkinson's Disease.

In one aspect the invention provides a screening assay with steps including a) exposing a plurality of test samples to thermal destabilization conditions, where each test sample contains i) parkin protein and ii) one of a plurality of test agents; b) determining parkin ligase activity in said test samples relative to a control sample comprising parkin protein exposed in the absence of a test agent to the thermal destabilization conditions, where a test agent contained in a test sample in which parkin ligase activity exceeds the ligase activity in the control sample is identified as a candidate compound for treatment of Parkinson's Disease. In one embodiment the parkin exposed in the absence of a test agent to the thermal destabilization conditions retains 40-70% of the its original E3 ligase activity. Examples of thermal destabilization conditions include incubation at a temperature of from 45°C to 60°C for 30 minutes to 180 minutes. For illustration, incubation can be at about 57°C for about 90 minutes or about 60°C for about 150 minutes.

In the assay, parkin ligase activity can be determined by combining parkin protein, an E1 ubiquitin-activating enzyme, an E2 ubiquitin-conjugating enzyme, ATP, ubiquitin, and a parkin substrate in an appropriate buffer, incubating the combination at 20-37°C and measuring the rate or extent of ubiquitination of the parkin substrate. Examples of parkin substrates are S5a (e.g., GST-S5a), septin 4, and troponin 1.

In the assay parkin ligase activity can be determined using a Fluorescence Resonance Energy Transfer (FRET) assay in which a donor chromophore is associated with ubiquitin and an acceptor chromophore is associated with a parkin substrate, or in which a donor chromophore is associated with parkin substrate and an acceptor chromophore is associated with a ubiquitin. In an embodiment the donor chromophore is europium cryplate and the acceptor chromophore is allophycocyanin. In an embodiment the parkin substrate is S5a. In a version of the assay candidate compounds are ranked according to the parkin ligase activity of the corresponding test sample.

Positive modulators of parkin activity that are parkin stabilizers may be distinguished from candidate compounds that are parkin agonists by incubating unattenuated parkin protein in the presence and absence of said compound, where a compound that increases parkin ligase activity is identified as a parkin agonist and a compound that does not increase parkin ligase activity is identified as a parkin stabilizer.

In one aspect the invention provides an in vitro method to assess the specificity of a positive modulator of parkin activity by (a) identifying a positive modulator of parkin using the assay of claim 1; (b) incubating an E3 ligase protein other than parkin and a parkin substrate protein together under conditions in which the substrate is ubiquitinated; (c) incubating the E3 ligase protein and the parkin substrate protein together in the presence of a positive modulator of parkin activity, under the conditions of (b); (d) comparing the ligase activity of the E3 ligase in the presence and absence of the positive modulator, where an increase in E3 ligase activity when the positive modulator is present indicates the positive modulator is not completely specific for parkin, and the absence of an increase indicates positive modulator is completely specific for parkin. In one version of the assay an increase in substrate ubiquitination in the presence of the positive modulator indicates the positive modulator is not completely specific for parkin, but positive modulator is partially specific where partial specificity is defined as an EC10 for the non-parkin E3 not more than 100 micromolar and is at least 4-fold higher than the EC10 for parkin.

Examples of parkin substrates for use in the assay include S5a and troponin 1. Examples of E3 ligase proteins in the assay include RING E3 ligases, Mdm2, Nedd4, Murfl, and E6AP.

In one aspect the invention provides a method for selecting a compound for treatment of Parkinson's Disease by (a) identifying positive modulators of parkin activity using the assay of claim 1; (b) identify positive modulators of (a) as parkin stabilizers or parkin agonists; (c) select positive modulators that are parkin specific based on the effect of the modulators on ubiquitination of a parkin substrate by an E3 ligase other than parkin (d) select positive modulators that are not substrate specific based on their ability to positively modulate parkin ubiquitination of more than one parkin substrate. In certain embodiments the parkin substrates include Septin 4, or Septin 4 and one or both of S5a or troponin 1.

### Brief Description of the Figure

Figure 1 is a diagram illustrating a TR-FRET assay for parkin stabilizers and agonists.

Figure 2 shows the results of a parkin ligase assay following thermal destabilization of parkin, in which the transition point for parkin thermal stability is shown to be between 42°C and 47°C.

Figure 3 shows results of a thermal denaturation assay.

Figure 4 shows is a graph showing the effect of a compound on parkin and mdm2 E3 ligase activity using S5a as substrate. The compound increased parkin activity with an EC50 of 2.8 uM but did not increase E3 ligase activity of mdm2.

### Detailed Description

Loss of parkin protein activity in humans results in the progressive loss of dopaminergic neurons in the *substantia nigra* and eventually to Parkinson's Disease. It has been discovered that agents that reverse, reduce, or prevent loss of parkin activity are candidate compounds for treatment and prevention of Parkinson's Disease. The present invention provides screening assays and other methods for identifying such agents.

Parkin protein is an E3 (ubiquitin) ligase. Parkin acts in conjunction with an E1 ubiquitin-activating enzyme and an E2 ubiquitin-conjugating enzyme to direct proteins to the ubiquitin/proteasome protein degradation pathway. The E1 enzyme uses ATP to activate ubiquitin for conjugation and transfers it to the E2 enzyme. Parkin interacts with the E2 enzyme and transfers the ubiquitin to a lysine ε-amino group on a target protein, thereby ubiquitinating the target protein. A polyubiquitin chain can be generated by consecutive addition of ubiquitin moieties to the ubiquitinated substrate. Parkin ligase activity can be assayed *in vitro* by measuring the rate or extent of ubiquitination.

In one aspect, a screening assay of the invention involves obtaining a plurality of test samples, each of which comprises parkin protein and one of a plurality of test agents, and exposing the test samples to thermal destabilization conditions *(i.e.,* elevated temperature sufficient to reduce ligase activity of native parkin protein). Parkin ligase activity is determined for each test sample to identify any test agent(s) that preserve or increase parkin activity relative to a control sample comprising parkin protein exposed to thermal destabilization conditions in the absence of a test agent. A test agent contained in a test sample in which parkin ligase activity exceeds the ligase activity in the control sample is identified as a candidate compound for treatment of Parkinson's Disease. These and other aspects of the invention are described in greater detail below.

### Test Samples

To identify agents that maintain parkin activity under destabilizing conditions, a plurality of test samples are exposed to an elevated temperature that, absent a test agent, reduces parkin ligase activity. As described below, each test sample contains parkin and a test agent in an appropriate medium. Without intending to be bound by a particular mechanism it is believed that some such agents stabilize parkin protein in its active conformation and/or catalyze renaturation ("stabilizers"), while others may increase the activity of native parkin ("agonists"). Advantageously, assays of the invention allow stabilizers and agonists to be distinguished from each other.

### i) Parkin

Parkin protein used in the assay most often has a sequence substantially the same as human parkin. An exemplary sequence for a human Parkin protein is found under NCBI accession number BAA25751 (see, *e.g.,* SEQ ID NO:1 and SEQ ID NO:2). Alternatively, parkin proteins from non-human mammals (*e.g.,* mouse) may be used. An exemplary sequence for a mouse Parkin protein is found under NCBI accession number AAI13205 (see, *e.g.,* SEQ ID NO:3 and SEQ ID NO:4). Parkin protein is typically obtained by recombinant expression using methods described widely in the scientific literature. Parkin may be produced in eukaryotic cell culture, in *E. coli* (*See, e.g.,* US 2007/0212679), or in other protein expression systems known in the art.

Parkin protein used in the assay may have the wild-type sequence or may be an allelic or other naturally occurring variant, or a recombinantly produced variant, that differs by a substitution, insertion, deletion of one or more residues, provided the protein retains at least some ligase activity. Recombinant parkin used in the assay is often modified to some degree. For example, in recombinantly expressed proteins (e.g., fusion proteins) there are often small changes (*e.g.,* deletion of the N-terminal methionine) of from 1-10 residues to facilitate expression. Parkin may be modified by addition of an epitope tag to facilitate production, detection or purification may be used. Common epitope tags for labeling proteins used in the present invention include FLAG, glutathione-S-transferase (GST), polyhistidine (His₆) (SEQ ID NO:5), Myc, maltose binding protein (MBP).

A parkin form suitable in the present assay will generally retain at least 50% of the ligase activity of the same molar amount of the wild-type human parkin, preferably at least 75%, often at least 80%, and most very at least 90%. Parkin fragments that retain ligase activity can be used. Typically such fragments comprise at least 400 contiguous residues of a naturally occurring Parkin sequence, and often at least 500 contiguous residues. In some embodiments variants of Parkin used in the present invention share at least 90% sequence identity, sometimes at least 95% sequence identity, and often at least 98% sequence identity with a naturally occurring form of parkin. Sequence identity between two proteins may be determined by optimally aligning the two protein sequences. Proteins can be aligned manually or using computer-implemented algorithms such as ClustalW and the NCBI alignment program, using default parameters.

Parkin variants associated with increased risk of Parkinson's Disease may also be used provided they retain at least some ligase activity. Examples of variants associated with increased risk include parkin having asparagine instead of serine at position 167; tyrosine instead of cysteine at position 212; methionine instead of threonine at position 240; tryptophan instead of arginine at position 275; glycine instead of cysteine at position 289; or leucine instead of proline at position 437.

### ii) Test agents

There is no particular limitation on the types of agents that can be screened for the ability to stabilize and/or activate parkin. For example, a number of natural and synthetic libraries of compounds can be used. *See, e.g.,* NCI Open Synthetic Compound Collection library, Bethesda, Md; Pirrung et al., 2008, "Synthetic Libraries of Fungal Natural Products" ChemInform 39:2; Shang et al., 2005, "Advancing chemistry and biology through diversity-oriented synthesis of natural product-like libraries" Curr Opin Chem Biol. 9:248-58; Webb TR, 2005, "Current directions in the evolution of compound libraries" Curr Opin Drug Discov Devel. 8:303-8; Fodor et al., 1991, Science 251:767-73; Medynski, 1994, BioTechnology 12:709-710; Ohlmeyer et al., 1993, Proc. Natl. Acad. Sci. USA 90:10922-26; Erb et al., 1994, Proc. Natl. Acad. Sci. USA 91:11422-26; Jayawickreme et al., 1994, Proc. Natl. Acad. Sci. USA 91:1614-18; and Salmon et al., 1993, Proc. Natl. Acad. Sci. USA 90:11708-712). The test agent can be a small molecule, such as a molecule with a molecular weight less than 1000, and often less than 500. Preferably the test agent can cross the blood-brain barrier or can be modified to a derivative that can cross the blood-brain barrier.

The concentration of test agent in the test sample will vary depending on the nature of the agent, but concentrations in the range of 1 nM to 5 µM are typical. Test agents may be prepared as a concentrated stock (*e.g.,* a 500 µM stock in DMSO or other appropriate buffer or solvent) prior to use in the screening assay. In the course of screening and validating a candidate compound, the effects on parkin activity of several different concentrations of a test agent may be measured (*e.g.,* 1 nM, 10 nM, 100 nM, 1 µM, 10 µM, 20 µM and 100 µM). In one embodiment, 10 µM and/or 20 µM test agent is used in the screening assay. Test agent concentrations refer to conditions at the time parkin protein is exposed to thermal destabilization conditions. It will be apparent from the discussion below that, following exposure to thermal destabilization conditions, reagents for measuring parkin activity are added to the test sample thereby increasing volume and reducing the concentration of test agent.

Generally the test agent is incubated with parkin during exposure to thermal destabilization conditions. In addition, the test agent may be pre-incubated with parkin under non-stress conditions (*e.g.,* 4°C to 37°C) for a period of minutes to hours (*e.g.,* 1 minute to 5 hours). Usually, at least some time lag between combining a test agent with parkin and exposure to thermal destabilization conditions required by the mechanics of reagent transfer (*e.g.,* pipetting). In one embodiment, a test agent is added to parkin protein at one temperature (*e.g.,* 4°C) and the test sample is subsequently incubated at a different temperature (*e.g.,* 37°C) for a period prior to exposure to the thermal destabilization conditions. In an alternative embodiment, a test agent is added to an already-attenuated parkin protein. In yet another alternative embodiment a test agent is added to an already-attenuated parkin protein and then exposed to further thermal destabilization.

Most often each test sample contains parkin protein and a single test agent. However, combinations of test agents can be included in a single test sample. Testing combinations can be useful, for example, for identifying additive or synergistic effects.

### Thermal destabilization conditions

Test samples containing parkin protein and test agents are exposed to thermal destabilization conditions, usually prior to initiation of the ligase reaction. Thermal destabilization conditions comprise a temperature and incubation time that results in thermal destabilization (or "attenuation") of parkin protein in the absence of a test agent. The thermal destabilization is detectable as a reduction in parkin ligase activity. Typical thermal destabilization conditions used in the assay reduce parkin ligase activity by about 10% - 100% compared to parkin protein not exposed to thermal destabilization (*e.g.,* maintained at 4°C for the duration of the assay). Often thermal destabilization conditions are selected that reduce parkin activity to about 40 - 80% of controls containing parkin not exposed to elevated temperature. Preferably conditions are selected that reduce parkin activity to about 40% to 70% of controls, such as 40-60% of controls. Parkin protein exposed to thermal destabilization conditions can be referred to as "attenuated parkin."

Optimal time and temperature parameters for parkin attenuation will vary somewhat with the buffer, concentration, test sample volume and form of parkin protein used. One suitable buffer for both the thermal destabilization and ligase assay steps is Assay Buffer A (50 mM HEPES pH 8.8, 1 mM DTT, 0.005% Tween^{®} 20 and 0.1 % Pluronic^{®} F-127). A second, and preferred buffer, Assay Buffer B (50 mM HEPES pH 8.8, 1 mM DTT, and 0.005% Tween^{®} 20). Parkin is typically exposed to thermal destabilization conditions at a concentration in the range 0.1 micrograms/ml to 10 mg/ml, although higher or lower concentrations may be used. Test sample volumes will depend on the format used and are often in the range of 50 nanoliters to 50 microliters, more often 500 nanoliters to 5 microliters. Test sample volumes may be lower, *e.g.,* in some microfluidic formats. In one embodiment thermal destabilization conditions are selected that reduce parkin activity by about 40 - 80% when measured using 0.1 mg/ml parkin in Assay Buffer A in a 500 nanoliter reaction volume.

Thermal destabilization of parkin is usually accomplished by exposure to temperatures above 45°C. As shown in Example 1, *infra,* the transition point for parkin thermal stability is in the range of 42 to 47°C. In experiments in which denaturation occurred in wells of 1,536-well microtiter plate somewhat higher temperatures were optimal. Generally the thermal denaturation conditions will comprise a temperature in the range of 45 to 60°C and an incubation time will be in the range of 30 minutes to 3 hours. Exemplary thermal destabilization conditions include 45-60°C for 30-120 minutes. For example, thermal denaturation can be carried out for 90 min at 57°C. In an other example, thermal destabilization conditions are 150 min at 60°C. In one embodiment parkin (0.5 mg/ml) and test agent (10 µM) are incubated in Assay Buffer A for 90 min at 57°C.

In addition to determining parkin activity in test samples containing parkin protein and a test agent, parallel determinations are carried out conducted with attenuated parkin in the absence of any test agent as well as parkin not exposed to thermal destabilization conditions. This is discussed further below, in the section captioned "*Reference samples*."

### Determining Parkin Ligase Activity: Materials, Formats and Methods

Following exposure to thermal destabilization condition, the parkin ligase activity in test (and reference) samples is determined. Assays for parkin ligase activity are known in the art, and a variety of formats and reagent combinations may be used in the screening methods of the present invention. It will be appreciated that the screening methods of the invention are not limited to any particular method for determining parkin activity.

The basic components of many parkin ligase assays are parkin protein, an E1 ubiquitin-activating enzyme (e.g., UBA1, UBA2), an E2 ubiquitin-conjugating enzyme (e.g., UbcH7, UbcH6, UbcH8, UbcH13), ATP (e.g., Mg-ATP), ubiquitin, a substrate (e.g., target protein), and an appropriate buffer or reaction medium. In one embodiment E1, E2, ATP, ubiquitin, a parkin substrate are added together to the test sample containing attenuated parkin to initiate the ligation reaction. Alternatively, the assay components may be added separately or sequentially. For example, E1, E2, ubiquitin, and a parkin substrate may be added to the test sample together, and the ligation reaction initiated by subsequent addition of ATP. In yet another variation, some assay components (*e.g.,* ATP) may be added prior to exposure to destabilizing conditions.

Assay components are commercially available (*e.g.,* Boston Biochem Inc., 840 Memorial Drive, Cambridge, MA 02139) and/or all may be obtained using methods known in the art or described below. *See, e.g.,* Wee et al., 2000, J. Protein Chemistry 19:489-98; and Zhang et al., 2000 Proc Natl Acad Sci U S A. 97:13354-9. Assay components may be purified and/or recombinant and may be human, mammalian, mouse or from other eukaryotes. In some versions of the assay, the components are derived from the same species (*e.g.,* parkin, S5a, E1, E2 and ubiquitin are all human, are all mouse, *etc.*).

An exemplary E1 ubiquitin-activating enzyme is UBA1 (Genbank accession No. X55386). Suitable E2 ubiquitin-conjugating enzymes include UbcH7, UbcH5, UbcH13 and UbcH13/Uev1. Parkin substrates that may be used in the assay include, but are not limited to, alpha-synuclein, Septin-4, the 26S proteasome subunit S5a, troponin 1, the putative G protein-coupled receptor Pael-R (Imai et al., 2001, Cell 105:891-902) and the parkin protein itself (autoubiquitination). Preferred substrates are S5a, troponin 1, and Septin-4.

S5a is a parkin substrate (see copending application No. 60/898,947, ). S5a is a multiubiquitin-binding protein that binds the poly-ubiquitin chain though its ubiquitin interaction motif. S5a is described in Ferrell et al., 1996, "Molecular cloning and expression of a multiubiquitin chain binding subunit of the human 26S protease" FEBS Lett. 381 (1-2), 143-148; Coux et al., "Structure and functions of the 20S and 26S proteasomes" Annu. Rev. Biochem. 65, 801-847 (1996); Wang et al., 2005, J Mol Biol. 348(3):727-39; van Nocker, 1996, Mol Cell Biol 16: 6020-28; Katzmann et al., 2002, Nat. Rev. Mol. Cell. Biol. 3:893; and Young et al., 1998, J. Biol. Chem. 273:5461. The sequence of human S5a has accession number NP_002801 in the NCI protein database (see, *e.g.,* SEQ ID NO:6 and SEQ ID NO:7).

The S5a substrate may be modified for use in assays. For example, S5a may be expressed as a fusion protein and may include, for example, an epitope tag such as GST or His₆. GST-tagged S5a can be purchased from BioMol, Inc. (Plymouth Meeting, PA). His₆-tagged S5a can be prepared as described in Walters et al., 2002 Biochemistry 41:1767-77. Truncated forms or fragments that retain the ability to be ubiquitinated by parkin may be used, typically comprising at least 200 contiguous residues of a naturally occurring S5a sequence, often at least 300 contiguous residues, often at least 350 contiguous residues, sometimes at least 370 contiguous residues. In some embodiments variants of S5a with at least 90% sequence identity to the naturally occurring human protein (NP_002801) are used, sometimes at least 95% sequence identity, and often at least 98% sequence identity. Sequence identity between two proteins may be determined by optimally aligning the two protein sequences. Proteins can be aligned manually or using computer-implemented algorithms such as ClustalW and the NCBI alignment program, using default parameters.

Troponin 1 is a subunit of troponin (see, PCT publication WO 2008/095126, ). Troponin binds to actin in thin myofilaments to hold the troponin-tropomyosin complex in place. Troponin 1 may be expressed as a fusion protein and may include, for example, an epitope tag such as GST or His₆. Troponin 1 is commercially available or can be prepared using well-known protocols. Truncated forms or fragments of troponin 1 that retain the ability to be ubiquitinated by parkin may be used, typically comprising at least 150 contiguous residues of a naturally occurring troponin sequence, often at least 180 contiguous residues, often at least 200 contiguous residues, sometimes at least 205 contiguous residues. In some embodiments variants of troponin 1 with at least 90% sequence identity to the naturally occurring human protein (NCl Protein Database Accession No. NP_000354; see, *e.g.,* SEQ ID NO:8) are used, sometimes at least 95% sequence identity, and often at least 98% sequence identity. Sequence identity between two proteins may be determined by optimally aligning the two protein sequences. Proteins can be aligned manually or using computer-implemented algorithms such as ClustalW and the NCBI alignment program, using default parameters.

Septin 4 ("Sept4") is a member of a conserved protein family with functions in cell division. Three splice variants of Septin 4 have been identified to date: Sept4var1 (NCBI accession number NP_004565), Sept4var2 (also known as "ARTS") (NP_536340) and Sept4var3 (NP_536341). Sept4var1 and Sept4var3 have the same sequence except Sept4var1 contains an additional 21 amino acids at the N-terminus. Sept4var2 (ARTS) shares sequence identity with variants 1 and 3 for residues 1-247 and then diverges in sequence for amino acids 247-274 (see Larisch et al., 2000, Nature Cell Biol 2:915-20 ). Also see Chance et al., 2006, "Inherited focal, episodic neuropathies: hereditary neuropathy with liability to pressure palsies and hereditary neuralgic amyotrophy" Neuromolecular Med. 8(1-2):159-74; Spiliotis et al., 2006 "Here come the septins: novel polymers that coordinate intracellular functions and organization" J Cell Sci. 119(Pt 1):4-10; Hall et al., 2004, "The pathobiology of the septin gene family" J Pathol. 204(4):489-505; . Sept4var3 has been shown to be a Parkin substrate (data not shown). See copending application No. 60/939,335 .

In assays of the invention, the Sept4 protein may be Sept4var3. Alternatively the Sept4 protein may be Sept4var1. Alternatively the Sept4 protein may be Sept4var2. Variants, fragments and mixtures of isoforms may also be used. Isoform 1 and isoform 3 of Sept4 differ only at 21 amino acid residues at the amino terminus and are believed to have equivalent interactions with Parkin. Sept4var2 (ARTS) has homology at the amino terminal 1-247 residues. Sept4var2 is ubiquitinated and co-immunoprecipitation experiments from neuronal cells demonstrated that Sept4var2 and Parkin interact with each other.

In some embodiments, truncated forms of Sept4 can be used with the methods of the present invention. For example, as demonstrated in the experimental examples below, Sept4 variants missing up to 117 amino acids from the N-terminus retain their ability to be ubiquitinated by Parkin and can, thus, be used in assays of the invention. In some embodiments, other variants of Sept4 can be used to practice the methods of this invention, *e.g.,* Sept4 variants that differ from by insertions, deletions or substitutions. Useful variants retain the property of being a parkin ubiquitination substrate, which can be tested using assays known in the art and described herein. Other variants of Sept4 that can be used in the present invention include variants that share 90% sequence identity, preferably at least 95% sequence identity, preferably at least 98% sequence identity with a Sept4 protein. Those of skill in the art can easily determine the homology a variant shares with the parental protein by optimally aligning the two protein sequences. Alignment programs such as ClustalW and the NCBI alignment program are exemplary programs that can be used for optimally aligning two proteins.

A Sept4 protein may be expressed as a fusion protein and may include, for example, an epitope tag to facilitate purification and/or binding to a substrate such as a microtiter well. For example, Ihara and colleagues use a baculoviral system to express histidine tagged Sept4 proteins cloned from human and mouse (Ihara et al., 2007, Neuron, 53:519-33).

Depending on the specific format of the assay, assay components may be labeled or modified. For example, ubiquitin and/or other components may be biotinylated, tagged, fluorescenated or complexed with another agent. For example, the Homogeneous Time-Resolved Fluorescence (HTRF) described below is carried out using biotinylated parkin substrate and ubiquitin complexed with europium cryptate.

As noted above, the parkin ligase reaction can be initiated by adding the assay reagents to a preparation containing attenuated parkin. It is convenient to prepare a "pre-mix" containing E1, E2, Mg-ATP, ubiquitin and substrate, in a suitable buffer or carrier (*e.g.,* Assay Buffer A). In one embodiment both the parkin attenuation step and the parkin ligase assay are carried out the same medium (*e.g.,* Assay Buffer A). The ligation reaction is allowed to proceed at a temperature in the range of 20 to 37°C (*e.g.,* room temperature, 30°C or 37°C) for, *e.g.,* 30 min to 4 hours. In one embodiment the ligation reaction is carried out at 30°C for 180 min.

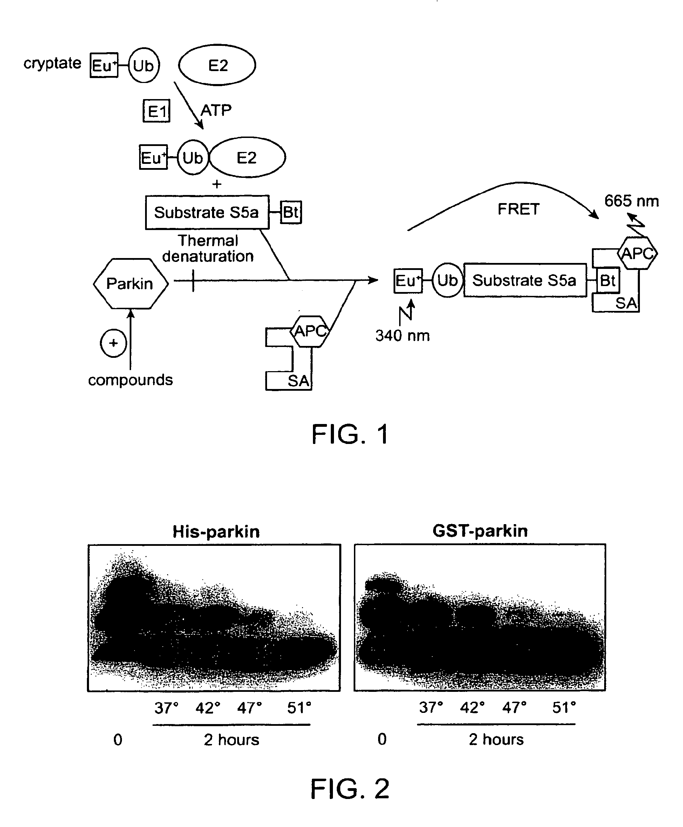

The rate or extent of ubiquitination of a substrate (*e.g.,* S5a) can be measured in a variety of ways. One method entails carrying out a ubiquitination reaction, separating proteins in the reaction mixture by electrophoresis, Western Blotting the separated proteins, probing the Western Blot with an anti-substrate (**e.g.,** anti-S5a) or anti-ubiquitin antibody, and detecting changes in mobility that reflect attachment of ubiquitin to the substrate. See Figure 2. However, any method of measuring ligase activity can be used, including immunologically based assays (ELISA, immunoprecipitation, see Harlow and Lane, 1988, ANTIBODIES, A LABORATORY MANUAL, Cold Spring Harbor Publications, New York, ), mass spectroscopic methods, electromagnetic spectrum spectroscopic methods, chromatographic methods, assays using detectably labeled ubiquitin, and Fluorescence Resonance Energy Transfer (FRET)-type assays. One preferred assay is a Fluorescence Resonance Energy Transfer (FRET-type) assay, an example of which is described below. In one embodiment parkin ligase activity is determined using a FRET assay in which a donor chromophore is associated with ubiquitin and an acceptor chromophore is associated with a parkin substrate or an acceptor chromophore is associated with ubiquitin and a donor chromophore is associated with a parkin substrate.

Assays can be designed to measure total ubiquitination per unit mass of substrate at a particular end point and/or to measure the extent of poly-ubiquitination of substrate molecules (i.e., the length of ubiquitin chains). Assays can be designed to measure ubiquitination at multiple time points to determine the level of ubiquitination per unit time ("rate" of ubiquitination), or under varying conditions.

The screening assay can be carried out in any of a variety of formats. For example, parkin can be exposed to thermal destabilization conditions and ligase activity assays in a microfuge tube. Preferably however, the assay is carried out in a format suitable for highthroughput screening (HTS). In one approach, multiwell plates are used, preferably in conjunction with automated (robotic) handling of reagents and samples. Multiwell plates microtiter plates are available in several format including 96-well plates, 384-well plates and 1,536-well plates. In another approach, microfluidic assay devices are used.

In the screening assay a plurality of test samples are screened simultaneously. The number of test agents screened in each assay is usually at least 20, more usually at least 50, preferably at least 100, and often at least 200, 300 or 400. A screening assay will often include multiple test samples with the same test agent (duplicates and/or at different concentration) as well as reference samples (described below). It will be appreciated that the assay of the invention can also be carried out using a smaller number of test agents, including a single test agent, particularly to validate or characterize a test agent putatively identified as a stabilizer or agonist.

### Reference samples

In addition to test samples, assays generally include reference, or "control," samples.

One reference sample contains parkin but no test agent and is otherwise processed in the same manner as test samples (including the attenuation step). The ligase activity of this reference provides is the baseline against which parkin ligase activity in test samples is compared. A test agent contained in a test sample in which parkin ligase activity exceeds the ligase activity in the reference sample is identified as a candidate compound for treatment of Parkinson's Disease.

A second type of reference sample contains unattenuated parkin and a test agent.

A third type of reference sample contains unattenuated parkin and no test agent. The parkin ligase activity of this reference sample is a positive control for the ligase assay.

Test agents that are stabilizers of attenuated parkin can be distinguished from agonists of parkin activity by comparing the ligase activity of the test sample and reference sample(s). An agonist will increase activity in the reference sample containing unattenuated parkin and the test agent (agonist) relative to unattenuated parkin alone. Thus, the invention also provides a method for distinguishing candidate compounds that are parkin stabilizers from candidate compounds that are parkin agonists by incubating unattenuated parkin protein in the presence and absence of said compound, wherein a compound that increases parkin ligase activity is identified as a parkin agonist and a compound that does not increase parkin ligase activity is identified as a parkin stabilizer. The aforementioned determination can be made concurrently with a primary screen in which test agents are incubated with attenuated parkin to identify candidate compounds and/or can be carried out as a separate step after candidate compound(s) have been identified. It will be appreciated that a single test agent may have both agonist and stabilizer activities.

### Fluorescence Resonance Energy Transfer (FRET) Type Assay

In one embodiment a Homogeneous Time-Resolved Fluorescence (HTRF) substrate-ubiquitination assay is used for screening. Such an assay is illustrated in Figure 1. As illustrated in the figure, test samples containing parkin protein and test agent are combined with a pre-mix containing biotinylated parkin substrate, E1, E2, ubiquitin spiked with a ubiquitin europium cryplate complex [Ub-Eu(K)], and Mg-ATP. In one embodiment the substrate is biotinylated S5a (Bt-S5a). The Ub-Eu(K), is transferred by E1 in the presence of ATP to E2 (Ub-Eu(K)-E2), which then holds Ub-Eu(K) in an energy-rich thiolester bond for transfer onto substrates. Ub-Eu(K) is transferred from E2 to the biotinylated substrate S5a (Bt-S5a). Allophycocyanin-labeled stretavidin (SA-APC) is added. If Ub-Eu(K) was transferred to Bt-S5A, Eu³⁺ and APC are brought into close proximity, permitting energy transfer between the two fluorescent labels. To measure Fluorescence Resonance Energy Transfer (FRET), Eu³⁺ is excited at a wavelength of 320 nm. Then, time-resolved fluorescence emission is detected at 685 nm. In order to normalize the FRET signal, time-resolved fluorescence emission is recorded at 615 nm (emission of Ub-Eu(K) at that wavelength) as well. The readout is calculated as follows: ratio = emission at 665 nm / emission at 615 nm x 10,000. In this assay only ubiquitin that has been transferred to the substrate will be detected. Free Ub-Eu(K) or Ub-Eu(K)-E2 will not lead to an assay signal. Advantageously, this assay allows screening for both stabilizers of attenuated parkin and parkin agonists. A further advantage of this type of assay is that the selectivity of any test compounds identified as hits can be characterized by replacing parkin by another E3 ligase. It will be recognized by those of skill guided by this disclosure that a variety of FRET-type assays may be used in the screening method of the invention, including, for example, variations in which different donor-acceptor pairs are used (e.g., Cy3/Cy5; also see Kainmüller and Bannwarth, 2006, Helvetica Chimica Acta 89:3056-70).

### Ligase Specificity Screens

Positive modulators identified using the thermal destabilization assays described above are further characterized using additional screening steps to determine their specificity for parkin. "Specificity" means that a positive modulator is not an agonist or stabilizer for multiple E3 ligases tested, but modulates parkin exclusively or more effectively than it modulates other E3 ligases.

As demonstrated below in Example 3, S5a is a substrate for several E3 ligases. The invention provides an *in vitro* method for determining specificity of a positive modulator of parkin activity on ligation of S5a by an E3 ligase other than parkin is assessed. In one embodiment the assay involves (1) incubating an E3 ligase protein other than parkin and S5a protein together under conditions in which the S5a protein is ubiquitinated; (2) incubating E3 protein and S5a protein in the presence of a parkin positive modulator together under the conditions of (1); (3) comparing the rate or extent of S5a ubiquitination in the presence of the parkin positive modulator with the rate or extent of S5a ubiquitination in the absence of the parkin positive modulator, where a relative increase in S5a ubiquitination in the presence of the parkin activity modulator indicates that the parkin activity modulator positively modulates the activity of the non-parkin E3 ligase activity (*e.g.,* is an agonist of the non-parkin E3 ligase). A parkin activity modulator that modulates parkin ubiquitination of S5a, but does not detectably modulate non-parkin E3 ubiquitination of S5a is identified as having *specific* parkin positive modulatory activity. *See* Example 4, below. The assay can also be used using a different parkin substrate, such as troponin 1, or any other parkin substrate that can be ubiquitinated in the presence of E1, E2, and other reaction components discussed above.

A parkin positive modulator that also modulates non-parkin E3 ubiquitination of S5a, but does so less effectively than it modulates parkin activity, is identified as having parkin positive modulatory activity that is *partially specific* for parkin. In this context "less effectively" means that amount (concentration) of the compound required to increase parkin activity by 10% ("EC₁₀") is greater for the non-parkin E3 than for parkin. In the assay, 100% is defined as the total activity of fully-active (*i.e.,* not attenuated or denatured) of the E3 ligase in the absence of the compound. An EC₁₀ for the non-parkin E3 that is more than 2-fold greater than for parkin shows partial specificity, provided the EC₁₀ for the non-parkin E3 is not more than 100 micromolar. Preferably the EC₁₀ is at least 5-fold, 10-fold, 20-fold, or 100-fold higher. Thus a compound that increases parkin activity from 100% to 110% at a concentration of 1 micromolar and increases non-parkin activity from 100% to 110% at a concentration of 25 micromolar shows partial specificity. In some versions of the assay the parkin and non-parkin E3 may be partially attenuated and have less than 100% of the activity of fully-active ligase. In such an example, a compound that increases attenuated parkin activity from 50% to 60% at a concentration of 1 micromolar and increases attenuated non-parkin activity from 55% to 65% at a concentration of 25 micromolar shows partial specificity. Dose-response curves can be generated using methods known in the art. Typically, serial 2-fold or 3-fold dilutions are used. Usually the concentrations tested are within the range 100 micromolar to 50 picomolar. A compound is considered completely specific of parkin if the EC₁₀ for the non-parkin E3 ligase(s) is greater than 100 micromolar and is at least 4-fold higher than the EC₁₀ for parkin.

The assay conditions for ligase activity of the non-parkin E3 may be, but are not necessarily, the same as those used in the parkin assay to which results are compared. For example, modifications may be made to account for differences among E3s in optimal reaction conditions or cofactors. For example, when the E3 is Mdm2 or Murf1 , the E2 protein may be UbcH5, while in a corresponding parkin assay a preferred E2 protein may be UbcH7. Specificity can be reported with reference to the assay reaction conditions and/or the non-parkin E3 ligase(s) tested. For example, the experiment described in Example 4 demonstrates that the compound tested is specific for parkin relative to Mdm2.

In cases in which thermal destabilization assays are used, E3 ligases are incubated under conditions of thermal denaturation, i.e., conditions that reduce E3 activity to about 30 - 80% of controls not exposed to elevated temperature. Conditions may be selected that reduce E3 activity to about 40% to 70% of controls, such as 40-60% of controls. E3 protein exposed to thermal destabilization conditions can be referred to as "attenuated E3." The conditions for denaturation will vary for different E3 ligases, but can be determined as described in the Examples. As is shown below, E3 E6AP appeared to lose 50% of its activity after pre-incubation for 1 hour at 41°C. E3 Murf1 appeared to lose 50% of its activity after pre-incubation for 1 hour at 60°C. It is within the skill of the practitioner guided by this disclosure to determine the thermal denaturation conditions for a given E3 ligase, that reduce activity by 40-70%.

Any mammalian E3 ligase that can ubiquitinate a parkin substrate can be used in a specificity assay using that substrate. For example, as shown in Example 4, CHIP, Nedd4, Murf1, E6AP, Mdm2 and Siah2 can ubiquitinate S5a. CHIP (carboxyl terminus of Hsp70-interacting protein) is a tetratricopeptide repeat-containing protein that interacts with heat shock proteins and negatively regulates chaperone functions (see, e.g., Ballinger et al., 1999, Mol. Cell. Biol. 19:4535-45; Connell et al., 2001, Nat. Cell Biol. 3: 93-96). Nedd4 (Neural precursor cell expressed developmentally down-regulated protein 4) is the prototypical protein in a family of E3 ubiquitin ligases that have a C2 domain at the N-terminus, two to four WW domains in the middle of the protein, and a catalytic HECT domain at the C-terminus (see, e.g., Ingham et al., 2004, Oncogene 23:1972-1984. Murf1 (Muscle-specific RING finger protein 1) is a protein critical in the development of muscle atrophy (see, e.g., Attaix et al., 2005, Essays Biochem. 41:173-186). Mdm2 (p53-binding protein Mdm2) is an oncoprotein that binds to the p53 tumor suppressor transactivation domain (Kussie et al., 1996, Science 274:948-953). E6AP (Human papillomavirus E6-associated protein) mediates the interaction of the human papillomavirus E6 oncoprotein with p53 (see Huibregtse et al., 1993, Mol. Cell. Biol. 13:775-784). Siah2 (Seven in absentia homolog 2) has been implicated in regulating cellular response to hypoxia (see, e.g., Nakayama et al., 2004, Cell 117:941-952). Other mammalian E3 ligases are readily identified by one of ordinary skill by reference to the medical literature. For illustration and not limitation examples include E3 ubiquitin ligase atrophin-interacting protein 4 (AIP4); EDD (or HYD); Smurf2; atrogin-1/MAFbx; RNF8;c-IAP1; SCf-Cdc4; Herc4; gp78; RINCK; Pirh2; Phr1; Triad1; RNF125/TRAC-1; Ufd2p; Ligand-of-Numb protein X1; Cullin4B; HRD-1; DDB2; BRCA1 RING; c-Cbl; HACK1; RNF5; Skp2; mind bomb 1; and Huwe1.

In some embodiments, the non-parkin E3 is a member of the RING family. In some embodiments the E3 ligase is selected from Mdm2, Nedd4, Murfl, and E6AP. In one embodiment the E3 ligase is Murf1 or E6AP. In one embodiment, the E3 ligase is Murf1. In some versions, the specificity assay uses a parkin substrate other than S5a, such as, for example, troponin 1.

### Drug Discovery Method

In one aspect the invention provides a method for positive modulators of parkin activity for use in therapy and prevention of Parkinson's Disease, having the steps shown in Table 1A or 1B.

**Table 1A**

| # | Action |
|---|---|
| 1 | Identify positive modulators of parkin activity based on increased ubiquitination of a parkin substrate (e.g., S5a, Septin4, troponin 1). |
| 2 | Select positive modulators of parkin activity that are parkin stabilizers. |
| 3 | Select positive modulators of parkin activity that are parkin specific based on the effect of the modulators on ubiquitination of a parkin substrate (e.g., S5a, troponin 1) by an E3 ligase other than parkin. |
| 4 | Select positive modulators that are not substrate specific (i.e., ubiquitinate more than one parkin substrate, e.g., ubiquitinate Septin4, S5a and troponin 1). |

**Table 1B**

| # | Action |
|---|---|
| 1 | Identify positive modulators of parkin activity based on increased ubiquitination of a parkin substrate (*e.g.,* S5a, Septin4, troponin 1). |
| 2 | Select positive modulators of parkin activity that are parkin agonists. |
| 3 | Select positive modulators of parkin activity that are parkin specific based on the effect of the modulators on ubiquitination of a parkin substrate by an E3 ligase other than parkin. |
| 4 | Select positive modulators that are not substrate specific (*i.e.,* ubiquitinate more than one parkin substrate, e.g., ubiquitinate Septin4, S5a and troponin 1). |

Steps 1-4 may be carried out in any order, provided Step 2-4 are carried out at the same time as or after Step 1. Step 4 is based on the discovery that Septin4 is a parkin substrate but is not a substrate for other E3 ligases. Step 1 is preferably carried out using the thermal denaturation assays described hereinabove. In some embodiments Step 2 is omitted. In some embodiments Steps 2 and 4 are omitted.

### Candidate compound

A test agent contained in a test sample in which parkin ligase activity exceeds the ligase activity in the reference sample is identified as a "hit" or candidate compound for treatment of Parkinson's Disease. Preferably a candidate compound (*e.g.,* a parkin stabilizer) will preserve at least 10% of the ligase activity lost due to thermal destabization. For example, if a reference sample with unattenuated parkin is defined as having 100% ligase activity, and a reference sample with attenuated parkin and no test agent has 50% of the ligase activity, a parkin stabilizer that preserves at least 10% of the ligase activity lost by thermal destabization will have at least 55% activity. More preferably a candidate compound will preserve at least 25% of the ligase activity, at least 30% of the ligase activity, at least 50% of the ligase activity, or at least 75% of the ligase activity lost due to thermal destabization.

Candidate agents may be ranked according to their ability to preserve parkin activity. The ranking may be recorded (*e.g.,* printed and/or stored on a computer-readable medium).

It will be understood that, as used herein, reference to an "agent useful for treating Parkinson's Disease" or "candidate compound for treatment of Parkinson's disease" refers to a compound identified as being more likely than other compounds to exhibit therapeutic or prophylactic benefit for patients with Parkinson's disease, *i.e.,* a drug candidate. It will be understood by those familiar with the process of drug discovery that a drug candidate may undergo further testing (e.g., *in vivo* testing in animals) prior to being administered to patients. It will also be understood that the agent approved for administration to humans may be a derivative of, or a chemically modified form of, the drug candidate.

For illustration, the lead compound may be modified to produce a prodrug form. For example, an ester linkage may be added to a lead compound to produce a pharmaceutically acceptable ester (*e.g.,* an ester that hydrolyzes under physiologically relevant conditions to produce a compound or a salt thereof) or a protecting group may be added to the compound. Illustrative examples of suitable ester groups include but are not limited to formates, acetates, propionates, butyrates, succinates, and ethylsuccinates. A variety of protecting groups are disclosed, for example, in T. H. Greene and P. G. M. Wuts, Protective Groups in Organic Synthesis, Third Edition, John Wiley & Sons, New York (1999). Conventional procedures for the selection and preparation of suitable prodrug derivatives are known in the art and described, for example, in "Design of Prodrugs," H. Bundgaard ed., Elsevier, 1985, and B. Testa "Hydrolysis in Drug and Prodrug Metabolism: Chemistry, Biochemistry, and Enzymology, 2003, Wiley-VCH.

Similarly, improvements in water solubility of a polyketide compound can be achieved by addition of groups containing solubilizing functionalities to the compound or by removal of hydrophobic groups from the compound, so as to decrease the lipophilicity of the compound. Typical groups containing solubilizing functionalities include, but are not limited to: 2-(dimethylaminoethyl)amino, piperidinyl, N-alkylpiperidinyl, hexahydropyranyl, furfuryl, tetrahydrofurfuryl, pyrrolidinyl, N-alkylpyrrolidinyl, piperazinylamino, N-alkylpiperazinyl, morpholinyl, N-alkylaziridinylmethyl, (1-azabicyclo[1.3.0]hex-1-yl)ethyl, 2-(N-methylpyrrolidin-2-yl)ethyl, 2-(4-imidazolyl)ethyl, 2-(1-methyl-4-imidazolyl)ethyl, 2-(1-methyl-5-imidazolyl)ethyl, 2-(4-pyridyl)ethyl, and 3-(4-morpholino)-1-propyl.

Many other modifications of compounds identified according to the invention will be apparent to those of skill, and can be accomplished using techniques of pharmaceutical chemistry.

Prior to use the candidate product (whether modified or not) can be formulated for storage, stability or administration. For example, the product can be formulated as a pharmaceutically acceptable salt. Suitable pharmaceutically acceptable salts of compounds include acid addition salts which may, for example, be formed by mixing a solution of the compound with a solution of a pharmaceutically acceptable acid such as hydrochloric acid, hydrobromic acid, sulfuric acid, fumaric acid, maleic acid, succinic acid, benzoic acid, acetic acid, citric acid, tartaric acid, phosphoric acid, carbonic acid, or the like. Where the compounds carry one or more acidic moieties, pharmaceutically acceptable salts may be formed by treatment of a solution of the compound with a solution of a pharmaceutically acceptable base, such as lithium hydroxide, sodium hydroxide, potassium hydroxide, tetraalkylammonium hydroxide, lithium carbonate, sodium carbonate, potassium carbonate, ammonia, alkylamines, or the like.

Prior to administration to a subject the product will be formulated as a pharmaceutical composition according to methods well known in the art, *e.g.,* combination with a pharmaceutically acceptable carrier. The term "pharmaceutically acceptable carrier" refers to a medium that is used to prepare a desired dosage form of a compound. A pharmaceutically acceptable carrier can include one or more solvents, diluents, or other liquid vehicles; dispersion or suspension aids; surface active agents; isotonic agents; thickening or emulsifying agents; preservatives; solid binders; lubricants; and the like. Remmington and Gennaro, 2006 Remington the science and practice of pharmacy. 21st Edition. Baltimore, Md, Lippincott Williams & Wilkins and Handbook of Pharmaceutical Excipients, Third Edition, A. H. Kibbe ed. (American Pharmaceutical Assoc. 2000), disclose various carriers used in formulating pharmaceutical compositions and known techniques for the preparation thereof.

The composition may be administered in any suitable form such as solid, semisolid, or liquid form. See Allen et al., (2005). Ansel's pharmaceutical dosage forms and drug delivery systems. Philadelphia, Lippincott Williams & Wilkins 8th Edition. In an embodiment, for illustration and not limitation, the polyketide is combined in admixture with an organic or inorganic carrier or excipient suitable for external, internal, or parenteral application. The active ingredient may be compounded, for example, with the usual nontoxic, pharmaceutically acceptable carriers for tablets, pellets, capsules, suppositories, pessaries, solutions, emulsions, suspensions, and any other form suitable for use. The carriers that can be used include water, glucose, lactose, gum acacia, gelatin, mannitol, starch paste, magnesium trisilicate, talc, corn starch, keratin, colloidal silica, potato starch, urea, and other carriers suitable for use in manufacturing preparations, in solid, semi-solid or liquified form. In addition, auxiliary stabilizing, thickening, and coloring agents and perfumes may be used.

### Example 1: Thermal Attenuation Parameters of Parkin Protein

Parkin attenuation (thermal destabilization) and *in vitro* assays were run in a 50ul eppendorf tube assay format, followed by Western blotting with antibodies to S5a to assess extent of ubiquitination of S5a by Parkin.

Parkin protein was pre-incubated for two hours at 37, 42, 47 and 51°C to thermally destabilize parkin protein. Two different parkin protein preparations were used. Preparation I ("His-parkin") is a histidine tagged parkin (described in US 2007/0212679). Preparation 2 ("GST-parkin") is a glutathione S-transferase tagged parkin. After thermal destabilization, Parkin was incubated on ice for ten minutes and then ligase assay reaction mix containing E1, E2 (UbcH7), S5a, Mg-ATP and ubiquitin was added.

Reaction mixtures were incubated for 90 min at 37°C and stopped with 4x Laemmli sample buffer, followed by immunoblotting with antibodies to S5a.

The results are shown in Figure 2. It is parkin preparations made using two different methods and having two different epitope tags identify the same thermal destabilization parameters, suggesting these experiments reveal an intrinsic temperature at which Parkin is not stably folded. The transition point for Parkin thermal stability is between 42°C and 47°C.

### Example 2. Validation of FRET Screen

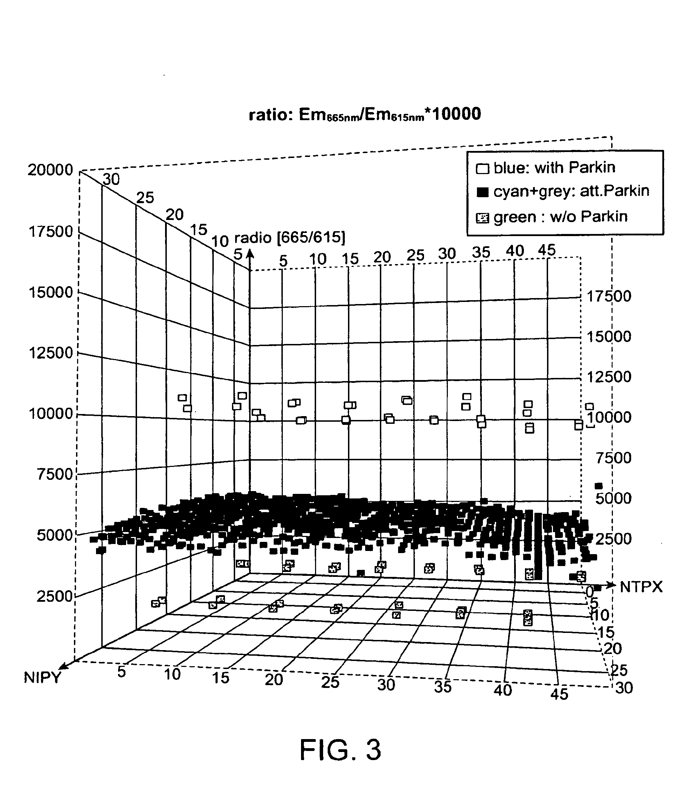

Figure 3 shows results of a thermal denaturation assay using the FRET format described above. A 1,536-well assay plate was used. In this experiment, test compounds were not included. The samples contained fully active parkin, attenuated parkin, or no parkin as indicated. The assay readout shows clear separation of the samples.

### Example 3: Ultra-High-Throughput-Screen (UHT screen) for Parkin Stabilizers and Agonists

Using a FRET-type assay of the invention 260,691 compounds were screened and 784 compounds were confirmed as candidate compounds for treatment of Parkinson's Disease. Parkin was thermally attenuated by exposure at 57°C for 90 min in the presence of test compounds. After attenuation, an assay reagent mixture (E1, E2, Eu-(K)-Ubiquitin, Mg-ATP, and biotinylated S5a) was added and incubated at 30°C to measure remaining Parkin activity. Ubiquitinylation of the biotinylated substrate, Bt-S5a, was determined by adding a streptavidin-conjugated acceptor cross linked allophycocyanin APC (XL-665, Cisbio Inc., Bedford, MA 01730) reagent and measuring the FRET signal between the Eu-(K)-Ubiquitin and the APC on the substrate. The assay is described in greater detail in the following paragraphs.

Thermal destabilization:
i) I uL of 0.05 mg/ml GST-Parkin in Assay Buffer A (50 mM Hepes pH 8.8, 1 mM DTT, 0.005% Tween^{®} 20 and 0.1% Pluronic^{®} F-127) was added to wells of a 1536-well plate.
ii) 10 or 20 nL test agent (500 uM stock in DMSO) was added test sample wells; Neat DMSO was added to reference sample wells.
iii) Samples were incubated for 90 min at 57°C.

Unattenuated parkin reference samples:
i) 1 uL of 0.05 mg/ml GST-Parkin in Assay Buffer A was added to wells of the 1,536-well plate (wells containing fully active parkin).

Ligation assay and detection:
i) 500 nL of a premix containing assay components was added to each well to a final concentration of:
   15 nM E1
   300 nM E2
   1 mM Mg-ATP
   400 nM Bt-S5a
   20 nM Ub-Eu(K)
   800 nM Ub
ii) The ligation (ubiquitinylation) reaction was allowed to proceed 180 min at 30°C.
iii) 3 uL of stop-detection mix was added to a final concentration of 75 nM streptavidin-conjugated XL665 (SA-XL665), 300 mM KF, 12 mM EDTA from a stock containing 100 mM NaPi pH 7.0, 100 nM SA-XL665, 400 mM KF, 16 mM EDTA, and 0.1% BSA.
iv) The 4 uL reaction mixture was incubation 45 min at room temperature.
v) HTRF read using an EnVision device (ParkinElmer) using the following parameters:
   a) Excitation at 320 nm.
   b) Emission at 665 nm & 615 nm measured.
   c) Delay time: 70 µs.
   d) Time window 100 µs.
   e) Time between flashes: 2000 µs.

Primary screen results
i) 4-5 replicates each of 260,691 compounds were screened at 20uM.
ii) The mean attenuation efficiency was 61 %.
iii) The hit threshold was the median 3*sigma of all plates (i.e., reference threshold was 18.62% activation) or the individual 3*sigma of the particular plate, whichever was higher.
iv) 3288 primary hit compounds were identified, reflecting a hit rate of 1.3%.

Confirmation screen results:
i) 4-5 replicates of each of the 3,288 primary hit compounds were tested.
ii) The mean attenuation efficiency was 67.4%.
iii) The reference hit threshold was 14.72%. Fewer than half the replicates must be above the threshold for a hit to be 'confirmed.'
iv) 784 compounds were confirmed as hits, reflecting a 24% confirmation rate.

### Example 4. Alternate E3 Ligases for Selectivity Screening

This example describes screening methods disclosed herein may be used to confirm the specificity of positive modulators of parkin activity. In this method the specificity of the positive modulator of parkin activity is determined by a) incubating an E3 ligase protein other than parkin and a parkin substrate protein together under conditions in which the substrate is ubiquitinated; (b) incubating the E3 ligase protein and the parkin substrate protein together in the presence of a positive modulator of parkin activity, under the conditions of (a); (c) comparing the ligase activity of the E3 ligase in the presence and absence of the positive modulator, where an increase in E3 ligase activity when the positive modulator is present indicates the positive modulator is not completely specific for parkin, and the absence of an increase indicates positive modulator is completely specific for parkin.

E3 ligases represent the largest family of ubiquitinating enzymes, with hundreds of putative sequences currently identified. There are three families of E3 ligases, grouped based on their structure and mechanism of action: (1) Homologous to E6AP Carboxy Terminus (HECT), (2) Really Interesting New Gene (RING) and (3) UFD2 homology (U-box). Assays of the invention may be, for example, a RING E3, a U-box E3, or a HECT E3. Parkin is a member of the RING family, and so it would be most valuable to utilize another RING family E3 as the alternate ligase for the compound screening. However, E3 ligases are historically challenging to express. Therefore we selected E3 ligases from each of the families to test for ability to ubiquitinate S5a. The ideal E3 ligase for use in secondary screens would express well, have high activity under the reaction conditions used in ubiquitination assays used for parkin, and can be thermally denatured under conditions similar to those used to disrupt parkin in thermal denaturation assays. Parkin has been discovered to have a denaturation temperature of 45-60°C. The ideal E3 ligase for specificity screening would have a thermal denaturation temperature in the range 45-60°C for use in the thermal stress assays developed for parkin screening.

We expressed and purified six E3 ligases (CHIP, Nedd4, Murfl, Mdm2, E6AP and Siah2) as described below (Section B). All of the E3 ligases were able to ubiquitinate S5a with high activity, with the exception of Siah2. Siah2 ubiquitinated S5a with very low activity. See Section C, below.

We then tested the ability of the E3 ligases to ubiquitinate S5a after pre-incubation at temperatures ranging from 4°C to 60°C. The thermal denaturation temperature was assessed for all E3s except CHIP and Siah2. See Section C, below. The temperature at which approximately 50% of activity was lost is listed in Table 2 for each E3 tested.

**Table 2**

| **Protein** | **E3 class** | **Expressed** | **Purified** | **Ubiquitinated S5a?** | **Thermal Denaturation Temperature** |
|---|---|---|---|---|---|
| GST-parkin | RING | Yes | Yes | Yes | 49°C |
| His-CHIP | U-box | Yes | Not well | Yes | N/A |
| GST-Nedd4 | HECT | Yes | Yes | Yes | 37°C |
| GST-Murf1 | RING | Yes | Yes | Yes | 60°C |
| GST-Mdm2 | RING | Yes | Yes | Yes | >60°C |
| GST-E6AP | HECT | Yes | Yes | Yes | 41°C |
| GST-Siah2 | RING | Yes | Yes | Yes (low) | N/A |

Based on these experiments we concluded that Nedd4, E6AP and Murf1 all showed good expression, purification and activity against S5a. CHIP expressed well and had reasonable activity for S5a, but was not a very pure sample. Siah2 was expressed and purified well, but showed very low activity for S5a. Thermal denaturation properties of Nedd4, E6AP, Murf1 and Mdm2 were assessed. Nedd4 was active after incubation at 37°C for 60 min, but lost activity by 90 min. Mdm2 had full activity even after pre-incubation at 60°C. E6AP showed thermal denaturation at 41°C and Murf1 showed thermal denaturation at 60°C.

Considering all of these results, the two most promising E3 ligases for use in thermal-denaturation based specificity screening were E6AP and Murf1. Since Murf1 is, like parkin, a RING E3, Murf1 is particularly well suited for the ligase secondary screen.

### A. Expression and Purification of E3 Lipases

Expression plasmids encoding GST fusions of the E3 ligases were transformed into BL21 DE3 pLysS cells and selected for based on ampicillin resistance. Cells were grown overnight in selective media and diluted 1:10 fold the following morning. When cell density reached the logarithmic phase of growth as measured by OD₆₀₀, expression was induced with 1mM IPTG. Expression differed in temperature and time and are listed in Table 3, below. Also provided are the types of affinity column used to purify the E3 protein and the final buffer in which the protein was dialyzed.

**Table 3**

| E3 | Protein Expression | | Affinity Column | Final Buffer |
|---|---|---|---|---|
| | temp | Time | | |
| His-CHIP | 25 | Overnight | Nickel | 50mM Tris pH 7.6, 10mM NaCl, 1mM DTT, 10% Glycerol |
| GST-Nedd4 | 16 | Overnight | GSH | 20mM Tris pH 7.6, 1mM DTT, 2mM EDTA, 20% Glycerol |
| GST-Murf1 | 25 | Overnight | GSH | 50mM Tris pH 7.6, 100mM NaCl, 1mM DTT, 10% Glycerol |
| GST-E6AP | 16 | Overnight | GSH | 20mM Tris pH 7.6, 1mM DTT, 2mM EDTA, 20% Glycerol |
| GST-Siah2 | 30 | 5 hrs | GSH | 50mM Tris pH7.6, 100mM NaCl, 1mM DTT, 10% Glycerol |

Expression and purification were monitored by PAGE using Coomassie staining to identify elution fractions containing the protein of interest.

### B. Ability to Use S5a as Substrate

For each E3 ligase, a ubiquitination assay using S5a as the substrate was carried out. Briefly, the ubiquitination reaction contained 50nM E1, 1mM MgATP, 5µM UbcH7, 0.2 mg/mL E3, 200nM ubiquitin, and 200nM S5a. Ubiquitination reactions were incubated for 1 hour at 37°C and samples were taken at time 0, 30' and 60'. Samples were run on SDS-PAGE, transferred to immobilon and Western blotted using monoclonal antibody to S5a (BioMol).

### C. Thermal Denaturation of E3 Ligases

To characterize the thermal denaturation properties E3 ligases, the E3 was pre-incubated for 90 minutes at a temperature ranging from 4°C to 60°C (Mdm2 and Nedd4 at 4, 37, 45, 50 and 60°C; Murf1 at 4, 37, 50, 60, 70 and 80°C; E6AP at 37, 39, 41, 43, and 45°C. At 90 minutes, a pre-mix was made containing 50nM E1, 1mM Mg-ATP, 5µM UbcH7 (UbcH5a for Mdm2 and Murfl), 200nM ubiquitin and 200nM S5a. The pre-mix was added to 0.2 mg/mL of E3 ligase and incubated at 37°C for 60 minutes. Samples were run on SDS-PAGE and assessed by Western blotting using monoclonal antibody to S5a (BioMol).

Parkin loses approximately 50% of its activity between 45°C and 50°C, which is consistent with earlier experiments. Mdm2 appears to retain activity even after pre-incubation at 60°C. Nedd4 appeared to lose activity following pre-incubation at any temperature except 4°C, under the conditions tested. E6AP appeared to lose 50% of its activity after pre-incubation at 41°C so we repeated this experiment using a range of temperatures from 37°C to 45°C in order to determine a more specific temperature at which E6AP undergoes thermal denaturation. Murf1 appeared to lose 50% of its activity at 60°C.

### Example 5. Ligase Selectivity Screening

Agents that inhibit or enhance parkin E3 ligase activity (hereinafter sometimes called "positive modulators") can be identified using assays in which S5a is the parkin substrate. Additional screening methods disclosed herein may be used to confirm the specificity of positive modulators for the parkin-S5a interaction. An agent that modulates parkin ubiquitination of S5a but does not modulate ubiquitination of S5a by a different E3 ligase is identified as having a positive modulatory activity specific for parkin.

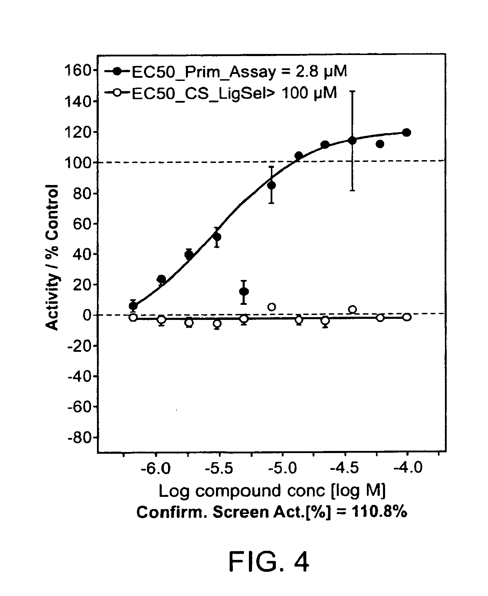

Figure 4 shows an experiment in which a positive modulator of parkin activity (EC₅₀ = 2.8 uM using GST-parkin PS/UbcH7) was tested for its effect on E3 ligase Mdm2. GST-Mdm2 was used at a concentration of 0.005 mg/ml with 100 nM UbcH5a in 1536-well format. As shown in the figure, the positive modulator of parkin activity did not increase activity of Mdm2, demonstrating that the positive modulator has specificity for parkin. This experiment did not use a thermal denaturation step.
Citation of publications and patent documents is not intended as an admission that any such document is pertinent prior art, nor does it constitute any admission as to the contents or date of the same. The invention having now been described by way of written description and example, those of skill in the art will recognize that the invention can be practiced in a variety of embodiments and that the foregoing description and examples are for purposes of illustration of the following claims.

## Claims

1. A screening assay comprising:
a) exposing a plurality of test samples to thermal destabilization conditions,
wherein each test sample comprises:
i) parkin protein; and
ii) one of a plurality of test agents; and
b) determining parkin ligase activity in said test samples relative to a control sample comprising parkin protein exposed in the absence of a test agent to the thermal destabilization conditions,
wherein a test agent contained in a test sample in which parkin ligase activity exceeds the ligase activity in the control sample is identified as a candidate compound for treatment of Parkinson's Disease.

2. The assay of claim 1 wherein parkin exposed in the absence of a test agent to the thermal destabilization conditions retains 40-70% of its original E3 ligase activity.

3. The assay of claim 2 wherein the thermal destabilization conditions comprise:
a) incubation at a temperature of from 45°C to 60°C for 30 to 180 minutes;
b) incubation at a temperature of about 57°C for about 90 minutes; or
c) incubation at a temperature of about 60°C for about 150 minutes.

4. The assay of claim 1 further comprising determining whether a candidate compound is a parkin stabilizer or a parkin agonist, comprising incubating unattenuated parkin protein in the presence and absence of said compound, wherein a compound that increases parkin ligase activity is identified as a parkin agonist and a compound that does not increase parkin ligase activity is identified as a parkin stabilizer.

5. The assay of claim 1 further comprising ranking said candidate compounds according to the parkin ligase activity of the corresponding test sample.

6. An in vitro assay to assess the specificity of a positive modulator of parkin activity comprising:
a) identifying a positive modulator of parkin activity using the assay of claim 1;
b) incubating an E3 ligase protein other than parkin and a parkin substrate protein together under conditions in which the substrate is ubiquitinated;
c) incubating the E3 ligase protein and the parkin substrate protein together in the presence of the positive modulator of parkin activity, under the conditions of (b); and
d) comparing the ligase activity of the E3 ligase in the presence and absence of the positive modulator, where an increase in E3 ligase activity when the positive modulator is present indicates the positive modulator is not completely specific for parkin, and the absence of an increase indicates the positive modulator is completely specific for parkin.

7. The in vitro assay of claim 6 wherein an increase in substrate ubiquitination in the presence of the positive modulator indicates the positive modulator is not completely specific for parkin, but the positive modulator is partially specific, wherein partial specificity is defined as an EC₁₀ for the non-parkin E3 that is not more than 100 micromolar and is at least 4-fold higher than the EC₁₀ for parkin.

8. The assay of any one of claims 1 to 7 wherein parkin ligase activity is determined by: (a) combining parkin protein, an E1 ubiquitin-activating enzyme, an E2 ubiquitin-conjugating enzyme, ATP, ubiquitin, and a parkin substrate in an appropriate buffer; (b) incubating the combination at 20-37°C; and (c) measuring the rate or extent of ubiquitination of the parkin substrate.

9. The assay of claim 8 wherein the parkin substrate is S5a, septin 4, or troponin 1.

10. The assay of any one of claims 6, 7 or 9 wherein the E3 ligase protein is selected from the group consisting of a RING E3 ligase, Mdm2, Nedd4, Murfl, and E6AP.

11. A method for selecting a compound for treatment of Parkinson's Disease comprising: (a) identifying positive modulators of parkin activity using the assay of claim 1 (b) identifying positive modulators of (a) as parkin stabilizers or parkin agonists; (c) selecting positive modulators that are parkin specific based on the effect of the modulators on ubiquitination of a parkin substrate by an E3 ligase other than parkin; and (d) selecting positive modulators that are not substrate specific based on their ability to positively modulate parkin ubiquitination of more than one parkin substrate.

12. The method of claim 11 wherein the more than one parkin substrate comprises Septin 4.

13. The method of claim 12 wherein the more than one parkin substrate comprises Septin 4 and one or both of S5a or troponin 1.

14. The assay of any one of claims to 7 wherein parkin ligase activity is determined using a fluorescence resonance energy transfer (FRET) assay in which a donor chromophore is associated with ubiquitin and an acceptor chromophore is associated with a parkin substrate, or in which a donor chromophore is associated with a parkin substrate and an acceptor chromophore is associated with ubiquitin, wherein the donor chromophore may be europium cryplate and the acceptor chromophore may be allophycocyanin.

## Patentansprüche

1. Screening-Test, bei dem man
a) mehrere Testproben thermischen Destabilisierungsbedingungen aussetzt, wobei die Testproben jeweils
i) Parkinprotein und
ii) eines von mehreren Testagentien enthalten, und
b) Parkin-Ligase-Aktivität in den Testproben relativ zu einer Parkinprotein enthaltenden Kontrollprobe, die den thermischen Destabilisierungsbedingungen in Abwesenheit eines Testagens ausgesetzt wurde, bestimmt,
wobei ein in einer Testprobe, bei der Parkin-Ligase-Aktivität höher als die Ligase-Aktivität in der Kontrollprobe ist, vorliegendes Testagens als eine Kandidatenverbindung für die Behandlung von Morbus Parkinson identifiziert wird.

2. Test nach Anspruch 1, wobei den thermischen Destabilisierungsbedingungen in Abwesenheit eines Testagens ausgesetztes Parkin 40-70% seiner ursprünglichen E3-Ligase-Aktivität behält.

3. Test nach Anspruch 2, wobei die thermischen Destabilisierungsbedingungen Folgendes umfassen:
a) 30 bis 180 Minuten Inkubation bei einer Temperatur von 45°C bis 60°C;
b) etwa 90 Minuten Inkubation bei einer Temperatur von etwa 57°C; oder
c) etwa 150 Minuten Inkubation bei einer Temperatur von etwa 60°C.

4. Test nach Anspruch 1, bei dem man ferner bestimmt, ob es sich bei einer Kandidatenverbindung um einen Parkin-Stabilisator oder einen Parkin-Agonisten handelt, wobei man nicht abgeschwächtes Parkinprotein in Gegenwart und Abwesenheit der Verbindung inkubiert, wobei eine Verbindung, die Parkin-Ligase-Aktivität erhöht, als ein Parkin-Agonist und eine Verbindung, die Parkin-Ligase-Aktivität nicht erhöht, als ein Parkin-Stabilisator identifiziert wird.

5. Test nach Anspruch 1, bei dem man ferner eine Rangliste der Kandidatenverbindungen gemäß der Parkin-Ligase-Aktivität der entsprechenden Testprobe aufstellt.

6. In-vitro-Test zur Beurteilung der Spezifität eines positiven Modulators der Parkin-Aktivität, wobei man
a) einen positiven Modulator der Parkin-Aktivität unter Verwendung des Tests nach Anspruch 1 identifiziert,
b) ein von Parkin verschiedenes E3-Ligaseprotein sowie ein Parkin-Substratprotein zusammen unter Bedingungen inkubiert, bei denen das Substrat ubiquitiniert wird,
c) das E3-Ligaseprotein und das Parkin-Substratprotein zusammen in Gegenwart des positiven Modulators der Parkin-Aktivität unter den Bedingungen von (b) inkubiert und
d) die Ligase-Aktivität der E3-Ligase in Gegenwart bzw. Abwesenheit des positiven Modulators vergleicht, wobei ein Anstieg der E3-Ligase-Aktivität bei Vorliegen des positiven Modulators anzeigt, dass der positive Modulator nicht vollkommen spezifisch für Parkin ist, und das Fehlen eines Anstiegs anzeigt, dass der positive Modulator vollkommen spezifisch für Parkin ist.

7. In-vitro-Test nach Anspruch 6, wobei ein Anstieg der Substratubiquitinierung in Gegenwart des positiven Modulators anzeigt, dass der positive Modulator nicht vollkommen spezifisch für Parkin, sondern teilspezifisch ist, wobei Teilspezifität als ein EC₁₀-Wert für das Nicht-Parkin-E3 definiert ist, der nicht mehr als 100 mikromolar beträgt und wenigstens um das 4-Fache höher liegt als der EC₁₀-Wert für Parkin.

8. Test nach einem der Ansprüche 1 bis 7, wobei Parkin-Ligase-Aktivität bestimmt wird, indem man (a) Parkinprotein, ein E1-Ubiquitin-Aktivierungs-enzym, ein E2-Ubiquitin-Konjugationsenzym, ATP, Ubiquitin und ein Parkin-Substrat in einem geeigneten Puffer zusammengibt, (b) die Kombination bei 20-37°C inkubiert und (c) die Geschwindigkeit oder das Ausmaß der Ubiquitinierung des Parkin-Substrats misst.

9. Test nach Anspruch 8, wobei es sich bei dem Parkin-Substrat um S5a, Septin 4 oder Troponin 1 handelt.

10. Test nach einem der Ansprüche 6, 7 oder 9, wobei das E3-Ligaseprotein aus der aus einer RING-E3-Ligase, Mdm2, Nedd4, Murf1 und E6AP bestehenden Gruppe ausgewählt ist.

11. Verfahren zur Auswahl einer Verbindung für die Behandlung von Morbus Parkinson, bei dem man (a) positive Modulatoren der Parkin-Aktivität unter Verwendung des Tests nach Anspruch 1 identifiziert, (b) positive Modulatoren aus (a) als Parkin-Stabilisatoren oder Parkin-Agonisten identifiziert, (c) positive Modulatoren auswählt, die aufgrund der Wirkung der Modulatoren auf die Ubiquitinierung eines Parkin-Substrats durch eine von Parkin verschiedene E3-Ligase parkinspezifisch sind, und (d) positive Modulatoren auswählt, die aufgrund ihrer Fähigkeit, Parkin-Ubiquitinierung von mehr als einem Parkin-Substrat positiv zu modulieren, nicht substratspezifisch sind.

12. Verfahren nach Anspruch 11, wobei das mehr als eine Parkin-Substrat Septin 4 umfasst.

13. Verfahren nach Anspruch 12, wobei das mehr als eine Parkin-Substrat Septin 4 sowie S5a oder bzw. und Troponin 1 umfasst.

14. Test nach einem der Ansprüche 1 bis 7, wobei Parkin-Ligase-Aktivität unter Verwendung eines Fluoreszenzresonanzenergietransfer-(FRET-)Tests bestimmt wird, bei dem ein Donorchromophor mit Ubiquitin und ein Akzeptorchromophor mit einem Parkin-Substrat assoziiert ist oder bei dem ein Donorchromophor mit einem Parkin-Substrat und ein Akzeptorchromophor mit Ubiquitin assoziiert ist, wobei es sich bei dem Donorchromophor um Europiumcryptat und bei dem Akzeptorchromophor um Allophycocyanin handeln kann.

## Revendications

1. Essai de criblage comprenant :
a) l'exposition d'une pluralité d'échantillons d'essai à des conditions de déstabilisation thermique, chaque échantillon d'essai comprenant :
i) de la protéine parkine ; et
ii) l'un d'une pluralité d'agents d'essai ; et
b) la détermination de l'activité parkine ligase dans lesdits échantillons d'essai par rapport à un échantillon témoin comprenant la protéine parkine exposée en l'absence d'un agent d'essai aux conditions de déstabilisation thermique,
dans lequel un agent d'essai contenu dans un échantillon d'essai dans lequel l'activité parkine ligase dépasse l'activité ligase dans l'échantillon témoin est identifié en tant que composé candidat pour le traitement de la maladie de Parkinson.

2. Essai de la revendication 1 dans lequel la parkine exposée en l'absence d'un agent d'essai aux conditions de déstabilisation thermique conserve 40 à 70 % de son activité ligase E3 originale.

3. Essai de la revendication 2 dans lequel les conditions de déstabilisation thermique comprennent :
a) l'incubation à une température de 45 °C à 60 °C pendant 30 à 180 minutes ;
b) l'incubation à une température d'environ 57 °C pendant environ 90 minutes ; ou
c) l'incubation à une température d'environ 60 °C pendant environ 150 minutes.

4. Essai de la revendication 1 comprenant en outre la détermination si un composé candidat est un stabilisant de parkine ou un agoniste de parkine, comprenant l'incubation de protéine parkine atténuée en présence et en l'absence dudit composé, dans lequel un composé qui augmente l'activité parkine ligase est identifié en tant qu'agoniste de parkine et un composé qui n'augmente pas l'activité parkine ligase est identifié en tant que stabilisant de parkine.

5. Essai de la revendication 1 comprenant en outre le classement desdits composés candidats en fonction de l'activité parkine ligase de l'échantillon d'essai correspondant.

6. Essai *in vitro* pour évaluer la spécificité d'un modulateur positif d'activité parkine comprenant :
a) l'identification d'un modulateur positif d'activité parkine en utilisant l'essai de la revendication 1 ;
b) l'incubation d'une protéine ligase E3 autre que la parkine et une protéine de substrat de parkine conjointement dans des conditions dans lesquelles le substrat est ubiquitiné ;
c) l'incubation de la protéine ligase E3 et la protéine de substrat parkine conjointement en présence du modulateur positif d'activité parkine, dans les conditions de (b) ; et
d) la comparaison de l'activité ligase de la ligase E3 en présence et en l'absence du modulateur positif, où une augmentation de l'activité ligase E3 lorsque le modulateur positif est présent indique que le modulateur positif n'est pas totalement spécifique pour la parkine, et l'absence d'augmentation indique que le modulateur positif est totalement spécifique pour la parkine.

7. Essai *in vitro* de la revendication 6 dans lequel une augmentation de l'ubiquitination de substrat en présence du modulateur positif indique que le modulateur positif n'est pas totalement spécifique pour la parkine, mais le modulateur positif est partiellement spécifique, une spécificité partielle étant définie comme une CE₁₀ pour la non-parkine E3 qui n'est pas supérieure à 100 micromolaires et est au moins 4 fois supérieure à la CE₁₀ pour la parkine.

8. Essai de l'une quelconque des revendications 1 à 7 dans lequel l'activité parkine ligase est déterminée par : (a) combinaison de protéine parkine, d'une enzyme d'activation d'ubiquitine E1, d'une enzyme de conjugaison d'ubiquitine E2, d'ATP, d'ubiquitine, et d'un substrat de parkine dans un tampon approprié ; (b) incubation de la combinaison à 20 à 37 °C ; et (c) mesure du taux ou degré d'ubiquitination du substrat de parkine.

9. Essai de la revendication 8 dans lequel le substrat de parkine est S5a, la septine 4, ou la troponine 1.

10. Essai de l'une quelconque des revendications 6 ou 9 dans lequel la protéine ligase E3 est choisie dans le groupe constitué d'une ligase E3 RING, Mdm2, Nedd4, Murf1, et E6AP.

11. Procédé pour sélectionner un composé pour le traitement de la maladie de Parkinson comprenant :
(a) l'identification de modulateurs positifs de l'activité parkine en utilisant l'essai de la revendication 1 ; (b) l'identification de modulateurs positifs de (a) en tant que stabilisants de parkine ou agonistes de parkine ; (c) la sélection de modulateurs positifs qui sont spécifiques à la parkine sur la base de l'effet des modulateurs sur l'ubiquitination d'un substrat de parkine par une ligase E3 autre que la parkine ; et (d) la sélection de modulateurs positifs qui ne sont pas spécifiques à un substrat sur la base de leur capacité à moduler positivement l'ubiquitination de parkine de plus d'un substrat de parkine.

12. Procédé de la revendication 11 dans lequel les plusieurs substrats de parkine comprennent la septine 4.

13. Procédé de la revendication 12 dans lequel les plusieurs substrats de parkine comprennent la septine 4 et l'un ou les deux de S5a ou la troponine 1.

14. Essai de l'une quelconque des revendications 1 à 7 dans lequel l'activité parkine ligase est déterminée en utilisant un essai de transfert d'énergie de résonance de fluorescence (FRET) dans lequel un chromophore donneur est associé à l'ubiquitine et un chromophore accepteur est associé à un substrat de parkine, ou dans lequel un chromophore donneur est associé à un substrat de parkine et un chromophore accepteur est associé à l'ubiquitine, dans lequel le chromophore donneur peut être du cryplate d'europium et le chromophore accepteur peut être l'allophycocyanine.
